# EUROPEAN PATENT SPECIFICATION

(11) **EP 1 119 538 B1**
(45) Date of publication and mention of the grant of the patent: **01.12.2004**
(21) Application number: 99947841.5
(22) Date of filing: 23.09.1999
(51) Int. Cl.: C07C 43/196, C07C 49/675, C07C 49/747, C07C 43/188, C07C 35/42, A61P 17/00, A61P 35/00, A61P 13/12, A61P 1/16, A61P 25/00

(54) **DERIVATIVES OF PHENANTRENE FOR MEDICINAL USE AND A PROCESS FOR THEIR PREPARATION**
PHENANTRENDERIVATE MIT MEDIZINISCHER ANWENDUNG SOWIE DESSEN HERSTELLUNGSVERFAHREN
DERIVES DE PHENANTHRENE A USAGE MEDICAL ET LEUR PROCEDE DE PREPARATION

(30) Priority: 23.09.1998 IT TO980808
(43) Date of publication of application: 01.08.2001
(73) Proprietor: Ammar, Khodor, 40100 Bologna (IT)
(72) Inventor: Ammar, Khodor, 40100 Bologna (IT)
(74) Representative: Eccetto, Mauro
(86) International application number: PCT/IT1999/000299
(87) International publication number: WO 2000/017143

(56) References cited:
- WO-A-97/02030
- B. E. CROSS ET AL: "A Novel Rearrangement of trans-Tetrahydroabietic Acid in Sulphuric Acid" JOURNAL OF THE CHEMICAL SOCIETY, CHEMICAL COMMUNICATIONS., no. 22, 1974, pages 930-931, XP002127927 CHEMICAL SOCIETY. LETCHWORTH., GB ISSN: 0022-4936

## Description

### TECHNICAL FIELD

The present invention relates to new active compounds derived from retinoic acid and suitable for the preparation of pharmaceutical compositions for medicinal use in general, and for use in dermatology, oncology and the treatment of kidney, liver and nervous system disorders; the invention also extends to pharmaceutical compositions containing such compounds, and to the relative preparation method.

### BACKGROUND ART

In the treatment of numerous types of tumors, a need is felt to reduce the use of chemotherapy which, though effective in combatting tumors, has extremely serious side effects. At present, this is done by combining chemotherapy with the use of retinoic acid and its derivatives, which are variously effective in preventing and curing tumors by inhibiting cell proliferation, inducing differentiation and apoptosis, and controlling the expression of oncogenes and genes which suppress the tumor. Positive results using retinoic acid have been obtained in particular in the treatment of acute promyelocytic leukemia (APL).

Unfortunately, however, retinoic acid and its known derivatives also have serious side effects, one of which being hypervitaminosis A, so that a need is felt for a compound which is equally effective therapeutically as retinoic acid, but has significantly less drastic side effects.

### DISCLOSURE OF INVENTION

It is therefore an object of the present invention to provide new compounds of similar or superior therapeutic effectiveness as compared with retinoic acid and its known derivatives, but which have significantly less drastic side effects.

According to the present invention, there is provided an active compound, in particular to be used as a medicament in general and specifically in dermatology, oncology, and the treatment of kidney, liver and nervous system disorders; the compound being characterized by formula (I): 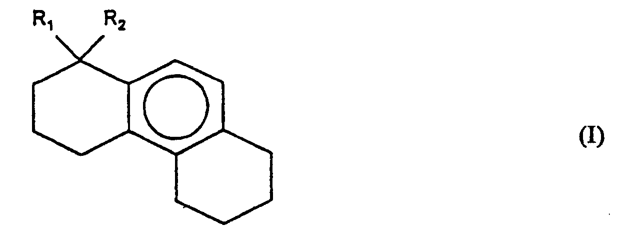 comprising three six-carbon condensed homocyclic rings including an aromatic central ring and two saturated or unsaturated aliphatic side rings in the meta position with respect to said aromatic central ring, and wherein the lateral substituents R, and R₂ are alkyl groups with a carbon number of 1 to 4; both said aliphatic side rings also possibly comprising one or more further lateral substituents selected from the group defined by: =O, -OH, -O-R', -O-R'-OH, where R' is a saturated or unsaturated straight- or branched-chain aliphatic group with a carbon number of 1 to 4.

More specifically, the compound according to the invention is characterized by formula (II): 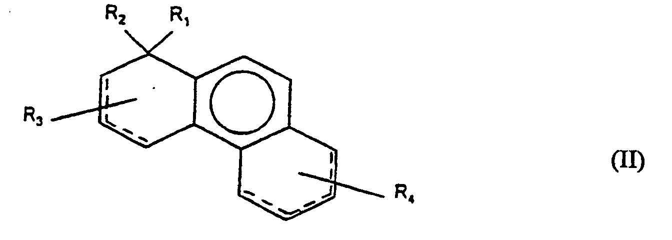 and comprises at least one of lateral substituents R₃ and R₄, and where:
- R₁ and R₂ are alkyl groups with a carbon number of 1 to 4;
- R₃ and R₄ are selected from the group defined by: =O, -OH, -O-R', -O-R'-OH, where R' is a saturated or unsaturated straight- or branched-chain aliphatic group with a carbon number of 1 to 4;
- the bonds represented by a continuous line alongside a dash line are double or single bonds.

The active compound according to the invention preferably has the formula (III): 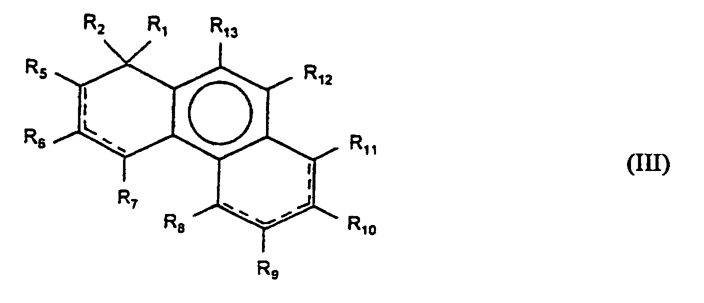 where:
- R₁ and R₂ are alkyl groups with a carbon number of 1 to 4;
- R₆, R₇, R₈, R₉, R₁₀, R₁₂, R₁₃ are selected independently from the group defined by: -H, -H₂, =O, -OH, -CH₃;
- R₅ and R₁₁ are selected from the group defined by; -H, -H₂, =O, -OH, -O-R', -O-R'-OH, where R' is a saturated or unsaturated straight- or branched-chain aliphatic group with a carbon number of 1 to 4;
- the bonds represented by a continuous line alongside a dash line are double or single bonds.

According to a preferred embodiment, R₁ and R₂ are methyl groups, and R₅ and R₁₁ are selected from the group defined by: -H, -H₂, =O, -OH, -O-CH₃, -O-CH=CH₂, -O-CH=CH₂-CH₃, -O-CH₂-CHOH-CH₃.

The present invention also relates to a pharmaceutical composition for medicinal use in general, and for use in dermatology, oncology, and the treatment of kidney, liver and nervous system disorders; characterized by comprising the compound defined above, in combination with pharmaceutically compatible excipients.

The present invention also relates to a method of preparing pharmaceutical compositions for medicinal use in general, and for use in dermatology, oncology, and the treatment of kidney, liver and nervous system disorders; characterized by comprising the steps of:
- preparing an alcoholic liquid solution of retinoic acid in an alcohol or glycol compound considerably in excess with respect to the retinoic acid;
- producing an esterification reaction of the retinoic acid with said excess alcohol or glycol compound at a predetermined temperature;
- allowing said reaction to proceed for a predetermined time at said predetermined temperature;
- interrupting said reaction after said predetermined time, and cooling said solution.

Said reaction is preferably produced by bringing said solution to the boil at a temperature of about 187°C. According to a preferred embodiment, said reaction is interrupted after about 60 minutes, and, according to a possible variation, is interrupted after about 120 minutes.

The therapeutic effect of the compounds according to the invention has been found to be substantially similar, if not superior, to that of retinoic acid in the treatment of leukemia, as demonstrated by research of cultured leukemic cell lineages.

Research, in particular of HL60 and U937 cell lineages, which are widely used in the study of leukemia on account of their close resemblance to leukemic cells, has shown the compounds according to the invention to be capable of differentiating the above lineages as effectively as, or even more effectively than, retinoic acid, and at lower concentrations, as confirmed by morphological and functional analyses. Above all, in the case of U937 cells, a significant increase was observed in the ability to produce thromboxane B2 which, in these cell lineages, represents a cell-differentiation-related index.

Moreover, the compounds according to the invention are nontoxic with regard to the cells, even when administered at relatively high (micromolar) concentrations for several consecutive days.

The compounds according to the invention also induce apoptosis in the cell lineages, as shown by the fall in the level ofbel-2 protein and by DNA fragmentation detected using various techniques : as is known, apoptosis, or programmed cell death, is an important biological process indispensable for normal cell development and differentiation, for maintaining homeostasis, and for virus or tumorous cell elimination by the host; and the tendency to induce apoptosis is an indication of effective therapeutic action.

Transfection analysis has shown the new compounds to interact preferentially with nuclear receptors of class RXR retinoids.

In short, in the cell lineages analyzed, the effects of the compounds according to the invention in terms of differentiation, reducing proliferation and inducing apoptosis are comparable, if not superior, to those of retinoic acid; which test results make it reasonable to assume such compounds will also be therapeutically effective in the treatment of leukemia and any other cases in which the effectiveness of retinoic acid has already been confirmed.

Numerous studies exist - including C. Chomienne et al., FASEB J., 10: 1025-1030 (1996) and La Press Medicale (1998) - confirming the effectiveness of retinoids (in particular retinoic acid) in arresting tumor development, by arresting the growth of neoplastic cells; and the similarity, observed in the cell lineages researched, between the behaviour of retinoic acid and the new compounds according to the invention makes it reasonable to assume the same also holds true for other applications of retinoic acid, such as the treatment of other types of tumors such as neuroblastomas.

In addition to oncology, the compounds according to the invention and the pharmaceutical compositions containing such compounds have also proved effective in other therapeutical sectors, in particular, dermatology and in the treatment of kidney, liver and nervous system disorders. More specifically, the compounds according to the invention have proved effective in all the cases in which the previous International Patent Application WO 97/02030 filed by the present Applicant indicated the possible use of glycol esters of retinoic acid.

### BEST MODE FOR CARRYING OUT THE INVENTION

A number of purely non-limiting embodiments of the present invention will now be described by way of example.

### EXAMPLE 1 : Preparation of the active compound

In a 2-liter vessel, a solution was prepared containing 2 g of retinoic acid in 300 ml of propylene glycol; the esterification reaction of the retinoic acid was initiated by adding 1 mg of salicylic acid as a catalyst and by bringing the solution to a (boiling) temperature of 187°C; the reaction was continued for an hour at the same temperature, and the resulting solution - referred to as solution n.1 - was cooled and added to an equal volume of distilled water.

The mixture was extracted in three successive stages using 150, 100 and 100 ml respectively of 1,1,2 trichloro-2,2,1 trifluoro-ethane in a separating funnel; the organic extract was then washed with 100 ml of a 0.1% aqueous solution of potassium bicarbonate (KHCO₃) and with 100 ml of distilled water to eliminate the catalyst.

The solvent was removed in a Rotavapor in a stream of nitrogen (known technique).

Solution n.1 was then analyzed to determine the compounds present. Elementary analysis showed the presence of about 70% of the crude formula compound C₂₁H₃₀O₃ of molecular weight 330; and spectrographic and, in particular, infrared analyses determined the following structural formula:

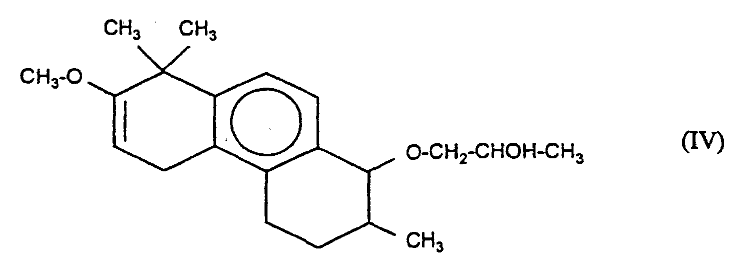

A second sample was prepared in the same way, but with a two-hour hot reaction between the retinoic acid and propylene glycol; and the resulting solution - referred to as solution n.2 - was purified and analyzed in the same way as solution n.1.

In addition to about 20% of the formula IV compound, solution n.2 was found to contain crude formula compounds C₁₇H₂₀O₂ and C₁₈H₂₄O of molecular weight 256 and C₁₇H₂₂O₂ of molecular weight 258, corresponding to the following formulas:

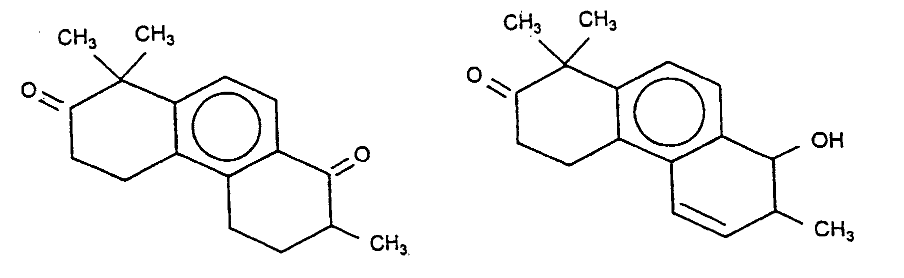

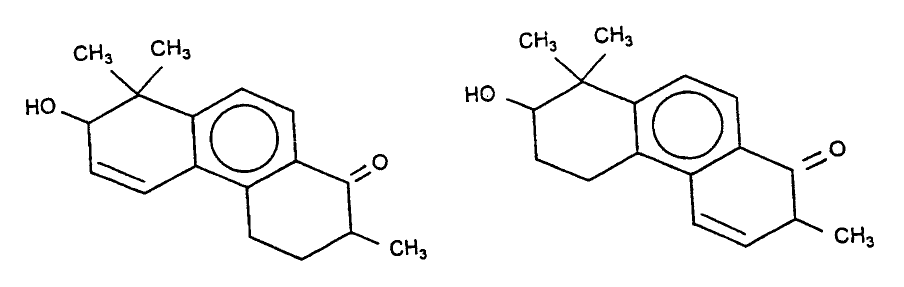

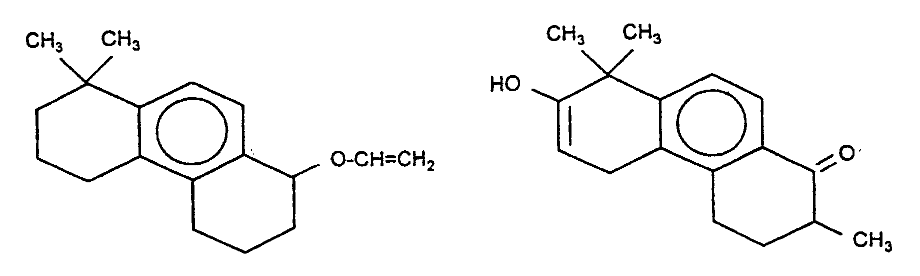

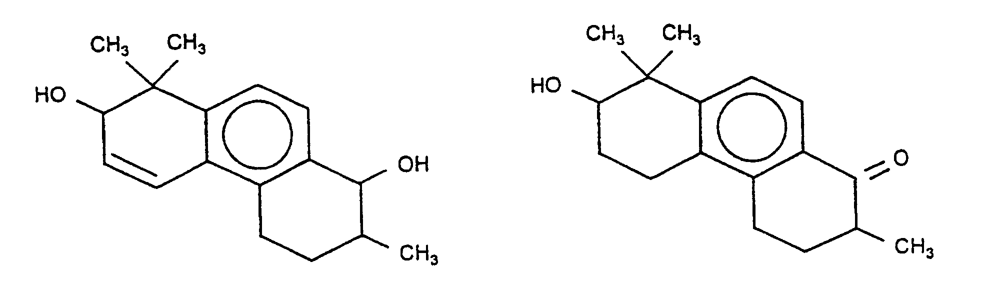

Solution n.2 was found to contain about 70% of isomers of molecular weight 256 and less than 10% of those of molecular weight 258.

Further compounds were prepared in the same way as described above, but using other alcoholic compounds, in particular ethanol, as opposed to propylene glycol for the hot retinoic acid reaction. The solutions obtained after one and two hours' reaction were found to contain further compounds similar to those in solutions n.1 and n.2, and all characterized by the presence of the group of the general formula:

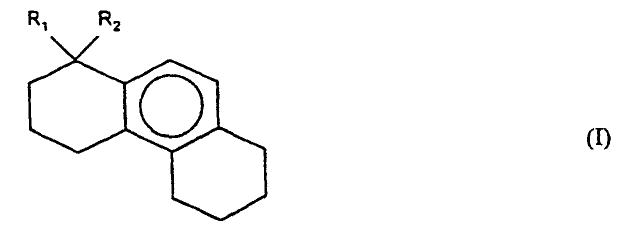

According to the invention, it is precisely the presence of this particular group - comprising three condensed hexagonal rings of carbon atoms, including an aromatic central ring and two saturated or unsaturated aliphatic rings in the meta position - which determines the therapeutic properties of the compounds, regardless of the nature and quantity of the lateral substituents.

### EXAMPLE 2: Therapeutic application

Both solution n.1 - mainly containing the formula IV compound (molecular weight 330) - and solution n.2 - mainly containing isomer compounds of molecular weight 256 - prepared as described in Example 1 were used to prepare pharmaceutical compositions for various therapeutic applications.

The active compounds according to the invention were used pure in low concentrations or diluted in aqueous glyco-alcohol solutions, possibly in combination with hydroquinone and/or salicylic acid.

Numerous patients suffering from dermatitis, psoriasis and various skin ailments were treated locally, with excellent results in all cases.

Administered orally, the compounds according to the invention also gave excellent results in the treatment of kidney ailments, cirrhosis of the liver, and nervous disorders.

In no cases were serious side effects recorded.

### EXAMPLE 3 : Effects on cultured leukemic cell lineages

Given the known differentiating and proliferation-retarding effects of retinoic acid on transformed cells, in particular leukemia cells U937 and HL60, tests were conducted to determine the same performance, and hence similar effects in the treatment of tumors, of the new retinoic-acid-derived compounds according to the present invention.

The results, using morphological; biochemical and biomolecular techniques, showed the new compounds according to the invention to induce differentiation of leukemia cells at lower concentrations as compared with retinoic acid, to be nontoxic with regard to the cells, even when administered in large doses for several consecutive days, and to be capable to inducing apoptosis in both the cell lineages considered (though in different ways), as shown by both morphological and functional analysis.

By way of further confirmation of the cell-maturing effect induced by the compounds, the reduction in proliferation in the cell lineages analyzed was found to be accompanied by a significant increase in the ability to produce thromboxane B2 (TXB2) which, as is known, in these cell lineages, is related to differentiation : for a given dosage, the production of TXB2 is about four times greater than that of cells treated with retinoic acid.

The compounds according to the invention were also found to have the same effects as retinoic acid in inducing apoptosis in leukemia cells, at least at given differentiation stages the ability of the compounds according to the invention to induce apoptosis in the cell lineages considered was determined in various ways using known techniques, both morphological (direct observation of slide specimens) and molecular (qualitative on gel and quantitative by diphenylation), and is demonstrated in particular by the fall in the level of bel-2 protein and by DNA fragmentation determined using various techniques.

Tests were also conducted to determine the effect of the compounds on cytoplasmic protein CRABP I and II and on nuclear protein RAR and RXR, the role played by which in the action of retinoic acid on cell mechanisms (though not yet fully explained) is by now confirmed. Transactivation tests conducted using known techniques have enabled definition of the receptor to which the compound according to the invention is preferentially bonded : test results have shown preferential bonding of the compounds to RXR class receptors, in particular RXR y, and significantly less affinity with RAR than with RXR class receptors. The compounds according to the invention therefore behave differently as compared with retinoic acid and its traditional derivatives, which, as is known, mainly bond with RAR receptors, whereas the compounds according to the invention preferentially bond with RXR receptors, to which are almost certainly bonded in particular those retinoids capable of inducing apoptosis in HL60 cells [36], in particular the 9-cis form of retinoic acid (the most effective in treating tumors).

Unlike retinoic acid, the compounds according to the invention appear not to induce CRABP II protein : this may be a sign of greater cell stability of the experimental compounds, the differentiating and apoptotic effects of which on transformed cells are thus prolonged.

In short, in the cell lineages considered, the activity of the new compounds according to the invention has proved comparable with, and in certain respects superior to, that of retinoic acid.

### EXAMPLE 4: Toxic activity

The compounds according to the invention were administered in various forms and concentrations to laboratory animals to determine toxicity.

A single-dose test was conducted using forty 21-week-old Warren stock chickens divided into four inoculation site groups : oral, subcutaneous, intramuscular, intravenous. Half the animals in each group were given 0.1 ml of product, and the other half 1 ml. Following administration of the product, the animals were kept under observation for 21 days to determine : general and/or local reactions, mortality, food and water consumption, and egg production.

A repeat-dose test was conducted in the same way using another forty 21-week-old Warren stock chickens, again divided into four inoculation site groups : oral, subcutaneous, intramuscular, intravenous. Half the animals in each group were given 0.1 ml of product, and the other half 1 ml; the same dose was repeated 15 days later; and, following the second dose, the animals were kept under observation for 21 days to determine the same parameters listed above.

Throughout the test period, none of the animals showed any clinical alteration or any change in standard zootechnical values : the mortality rate was zero, and no negative effects, either local or in zootechnical parameters, were observed. In short, in the dosages used, the product was found to be harmless and to have no local or overall negative effects.

### EXAMPLE 5: Activity on tumorous cells

Further research has recently been carried out using retinoic acid, sodium butyrate and the invention substances obtained as in Example 1. More specifically, tests were conducted on intestinal adenocarcinoma cells (lineages CACO-2 and HT-29) using isomers of molecular weight 256 - hereinafter referred to simply as "esters 256" - and using the compound of molecular weight 330 corresponding to formula IV, and mixtures mainly containing said compound - hereinafter referred to simply as "HHP" (hydroxy-hydro-phenanthrene). The results were as follows:
1. The proliferation-inhibiting effect of HHP and butyrate on cell lineage CACO-2 is 80% as compared with 60% for retinoic acid. Inhibition in cell lineage HT-29 treated with HHP of 50µM concentration was 95% by the seventh day.
2. HHP is therefore more active on the cells with a lower degree of differentiation.
3. HHP has the same effect as, but none of the side effects of, butyrate, whereas retinoic acid has a lesser and retarded effect.
4. A combination of HHP of 50 µM concentration and butyrate of 5 µM concentration is fairly promising in lineage CACO-2, but only brings about improvements in lineage HT-29 with smaller doses of butyrate.
5. HHP induces in the cell lineages a 3.5 times increase in the number of domes, indicating greater cell differentiation.
6. HHP increases the quantity of ALP (alkaline phosphatase) and is dose-dependent.
7. In CACO-2, alpha 2,6, xyalyl transferase increases 3 times with respect to the control after two weeks' treatment with HHP.

Further research has shown HHP only increases differentiation. and not apoptosis. HHP was tested on neuroblastoma cells, lineage TS-12; and was found to be more active than retinoic acid in terms of cell differentiation, and to have the same effect as sodium butyrate.

HHP was also found to reduce somatotropin with a reduction in PSA and a noticeable reduction in prostatic hypertrophy (37 to 27 in one month); to have a regulating effect on estrogen-progesterone with a reduction in estrogens; and to arrest angiogenesis of tumorous cells without affecting healthy blood vessels.

Large doses (2 g/day) of HHP were administered with no side effects. When administered to animals - horses and dogs, even of advanced age - HHP brought about an improvement in running speed and resistance to fatigue, and the animals ate and drank more than usual.

The drug may be administered diluted in oil to eliminate the propylene glycol.

## Claims

1. A compound **characterized by** formula (I): 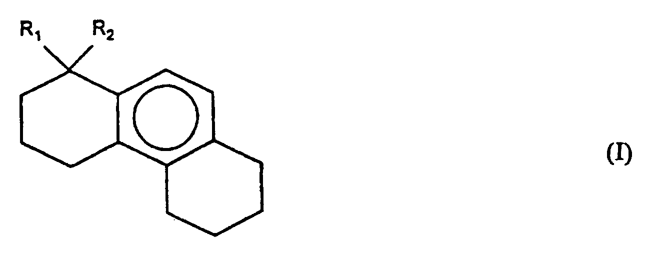 comprising three six-carbon condensed homocyclic rings including an aromatic central ring and two saturated or unsaturated aliphatic side rings in the meta position with respect to said aromatic central ring, and wherein the lateral substituents R₁ and R₂ are alkyl groups with a carbon number of 1 to 4; both said aliphatic side rings also possibly comprising one or more further lateral substituents selected from the group defined by: =O, -OH, -O-R', -O-R'-OH, where R' is a saturated or unsaturated straight- or branched-chain aliphatic group with a carbon number of 1 to 4.

2. A compound as claimed in the foregoing Claim, **characterized by** formula (II): 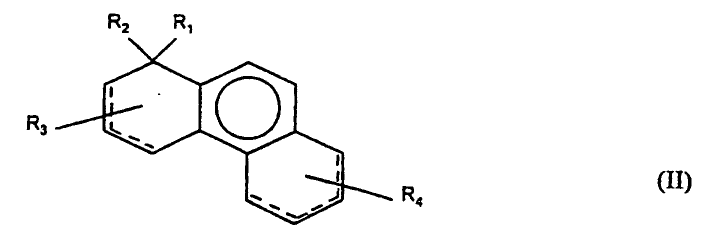 and comprising at least one of lateral substituents R₃ and R₄, and where:
- R1 and R2 are alkyl groups with a carbon number of 1 to 4;
- R3 and R4 are selected from the group defined by: =O, -OH, -O-R', -O-R'-OH, where R' is a saturated or unsaturated straight- or branched-chain aliphatic group with a carbon number of 1 to 4;
- the bonds represented by a continuous line alongside a dash line are double or single bonds.

3. A compound as claimed in the foregoing Claim, **characterized by** formula (III): 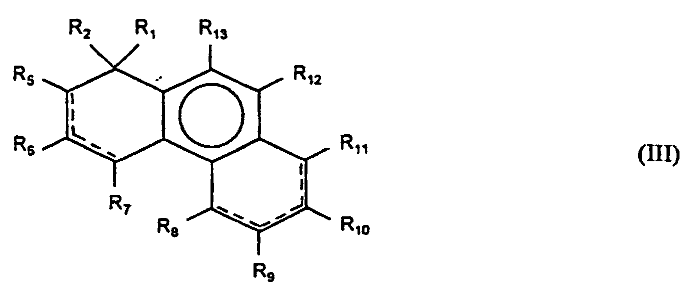 where:
- R₁ and R₂ are alkyl groups with a carbon number of 1 to 4;
- R₆, R₇, R₈, R₉, R₁₀, R₁₂, R₁₃ are selected independently from the group defined by:
- H, -H₂, =O, -OH, -CH₃;
- R₅ and R₁₁ are selected from the group defined by: -H, -H₂, =O, -OH, -O-R', -O-R'-OH, where R' is a saturated or unsaturated straight- or branched-chain aliphatic group with a carbon number of 1 to 4;
- the bonds represented by a continuous line alongside a dash line are double or single bonds.

4. A compound as claimed in the foregoing Claim, **characterized in that** R₁ and R₂ are methyl groups -CH₃, and R₅ and R₁₁ are selected from the group defined by: -H, -H₂, =O, -OH, -O-CH₃, -O-CH=CH₂, -O-CH=CH₂-CH₃, -O-CH₂-CHOH-CH₃.

5. A pharmaceutical composition for medicinal use in general, and for use in dermatology, oncology, and the treatment of kidney, liver and nervous system disorders; **characterized by** comprising the compound as claimed in any one of the foregoing Claims, in combination with pharmaceutically compatible excipients.

6. A composition as claimed in the foregoing Claim, **characterized by** also comprising hydroquinone and/or salicylic acid.

7. A method of preparing pharmaceutical compositions for medicinal use in general, and for use in dermatology, oncology, and the treatment of kidney, liver and nervous system disorders comprising the active compound as claimed in the foregoing claims 1-4 **characterized by** comprising the steps of:
- preparing an alcoholic liquid solution of retinoic acid in an alcohol or glycol compound considerably in excess with respect to the retinoic acid;
- producing an esterification reaction of the retinoic acid. with said excess alcohol or glycol compound at a predetermined temperature;
- allowing said reaction to proceed for a predetermined time at said predetermined temperature;
- interrupting said reaction after said predetermined time, and cooling said solution.

8. A method as claimed in the foregoing Claim, **characterized in that** said reaction is produced by bringing said solution to the boil at a temperature of about 187°C.

9. A method as claimed in Claim 7 or 8, **characterized in that** said reaction is interrupted after about 60 minutes.

10. A method as claimed in Claim 7 or 8, **characterized in that** said reaction is interrupted after about 120 minutes.

11. A compound as claimed in any one of Claims 1 to 4 for use as a medicament.

12. Use of a compound as claimed in any one of Claims 1 to 4 for the manufacture of a medicament for treatment of dermatological diseases.

13. Use of a compound as claimed in any one of Claims 1 to 4 for the manufacture of a medicament for treatment of tumorous cells.

14. Use of a compound as claimed in any one of Claims 1 to 4 for the manufacture of a medicament for treatment of kidney disorders.

15. Use of a compound as claimed in any one of Claims 1 to 4 for the manufacture of a medicament for treatment of liver disorders.

16. Use of a compound as claimed in any one of Claims 1 to 4 for the manufacture of a medicament for treatment of nervous system disorders.

## Patentansprüche

1. Verbindung, **gekennzeichnet durch** die Formel (I) 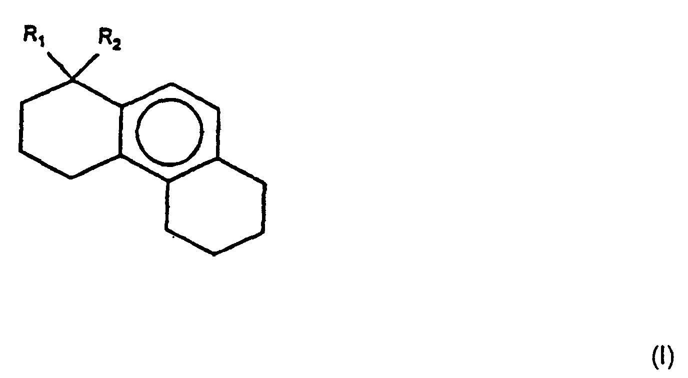 enthaltend drei kondensierte homozyklische Kohlenstoff-Sechsringe, umfassend einen mittleren aromatischen Ring und zwei gesättigte oder ungesättigte aliphatische seitliche Ringe, die in Metaposition zum mittleren aromatischen Ring angeordnet sind, wobei es sich bei den seitlichen Substituenten R₁ und R₂ um Alkylgruppen mit einer Kohlenstoffzahl von 1 bis 4 handelt; wobei beide aliphatischen Seitenringe zudem möglicherweise einen oder mehr zusätzliche seitliche Substituenten umfassen, die zu der **durch** =O, -OH, -O-R', -O-R'-OH definierten Gruppe gehören, und wobei es sich bei R' um eine gesättigte oder ungesättigte aliphatische Gruppe mit linearer oder verzweigter Kettenstruktur und einer Kohlenstoffzahl von 1 bis 4 handelt.

2. Verbindung nach dem vorhergehenden Anspruch, die durch die Formel (II) 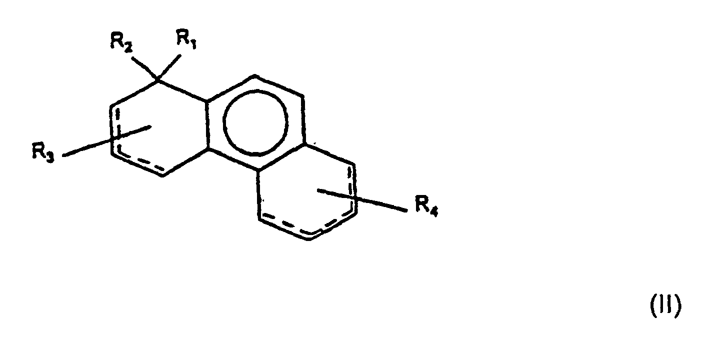 gekennzeichnet ist und wenigstens einen der seitlichen Substituenten R₃ und R₄ umfasst, wobei:
- es sich bei R1 und R2 um Alkylgruppen mit einer Kohlenstoffzahl von 1 bis 4 handelt; und
- R3 und R4 zu einer durch =O, -OH, -O-R', -O-R'-OH definierten Gruppe gehören, wobei R' für eine gesättigte oder ungesättigte aliphatische Gruppe mit linearer oder verzweigter Kettenstruktur und mit einer Kohlenstoffzahl von 1 bis 4 steht;
- wobei es sich bei den durch eine durchgehende Line neben einer gestrichelten Linie angedeuteten Bindungen um Doppelbindungen oder einfache Bindungen handelt.

3. Verbindung nach dem vorhergehenden Anspruch, **gekennzeichnet durch** die Formel (III) 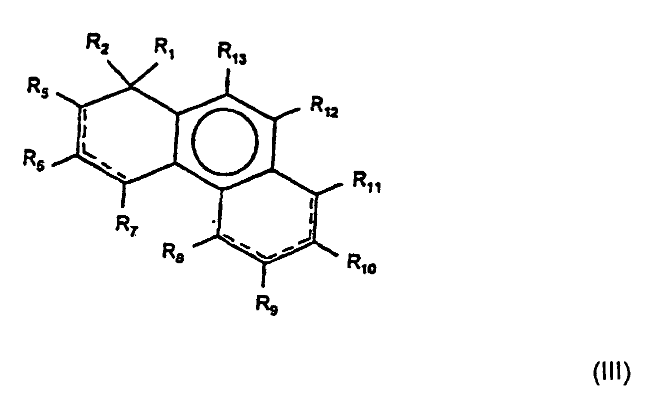 wobei
- es sich bei R₁ und R₂ um Aklylgruppen mit einer Kohlenstoffzahl von 1 bis 4 handelt;
- R₆, R₇, R₈, R₉, R₁₀, R₁₂, R₁₃ unabhängig voneinander aus der **durch** - H, -H₂, =O, -OH, -CH₃ definierten Gruppe ausgewählt werden,
- R₅ und R₁₁ aus der **durch** -H, -H₂, =O, -OH, -O-R', -O-R'-OH definierten Gruppe ausgewählt wurden, wobei es sich bei R' um eine gesättigte oder ungesättigte aliphatische Gruppe mit linearer oder verzweigter Kettenstruktur und einer Kohlenstoffzahl von 1 bis 4 handelt;
- die **durch** eine durchgehende Linie neben einer gestrichelten Linie dargestellten Bindungen Doppelbindungen oder einfache Bindungen sind.

4. Verbindung nach dem vorhergehenden Anspruch, **dadurch gekennzeichnet, dass** R1 und R2 Methylgruppen -CH₃ repräsentieren und R₅ und R₁₁ aus der durch -H, -H2, =O, -OH, -O-CH₃, -O-CH=CH₂, -O-CH=CH₂-CH3, -O-CH₂-CHOH-CH₃ definierten Gruppe ausgewählt werden.

5. Pharmazeutische Zusammensetzung für einen medizinischen Zweck im allgemeinen, und insbesondere für einen Einsatz in der Dermatologie, Onkologie bzw. bei der Behandlung von Erkrankungen der Nieren, der Leber und des Nervensystems; **dadurch gekennzeichnet, dass** die Zusammensetzung eine Verbindung nach einem der vorhergehenden Ansprüche in Kombination mit pharmazeutisch kompatiblen Arzneiträgem umfasst.

6. Zusammensetzung nach dem vorhergehenden Anspruch, **dadurch gekennzeichnet, dass** die Zusammensetzung zusätzlich Hydrochinon und/oder Salycilsäure enthält.

7. Verfahren zur Herstellung von pharmazeutischen Zusammensetzungen für medizinische Zwecke im allgemeinen und für den Einsatz in der Dermatologie, Onkologie und bei der Behandlung von Erkrankungen der Nieren, der Leber und des Nervensystems, enthaltend eine aktive Verbindung nach den vorhergehenden Ansprüchen 1 bis 4, wobei das Verfahren durch die folgenden Verfahrensschritte gekennzeichnet ist:
- Zubereitung einer alkoholischen flüssigen Lösung von Retinolsäure in einer relativ zur Retinolsäure erheblich im Überschuss vorhandenen Alkohol- oder Glykolverbindung,
- Hervorrufung einer Veresterungsreaktion der Retinolsäure mit der im Überschuss vorhandenen Alkohol- oder Glykolverbindung bei einer festgelegten Temperatur,
- Ermöglichung eines Fortschreitens dieser Reaktion über einen festgelegten Zeitraum bei der festgelegten Temperatur;
- Unterbrechen der Reaktion nach dem festgelegten Zeitraum und Kühlen der Lösung.

8. Verfahren nach einem der vorhergehenden Ansprüche, **dadurch gekennzeichnet**, das die Reaktion hervorgerufen wird, indem die Lösung bei einer Temperatur von etwa 187°C zum Sieden gebracht wird.

9. Verfahren nach Anspruch 7 oder 8, **dadurch gekennzeichnet, dass** die Reaktion nach etwa 60 Minuten unterbrochen wird.

10. Verfahren nach Anspruch 7 oder 8, **dadurch gekennzeichnet, dass** die Reaktion nach etwa 120 Minuten unterbrochen wird.

11. Verbindung nach einem der Ansprüche 1 bis 4 zum Einsatz als ein Medikament.

12. Einsatz einer Verbindung nach einem der Ansprüche 1 bis 4 zur Herstellung eines Medikaments für die Behandlung dermatologischer Erkrankungen.

13. Einsatz einer Verbindung nach einem der Ansprüche 1 bis 4 für die Herstellung eines Medikaments zur Behandlung von Tumorzellen.

14. Einsatz einer Verbindung nach einem der Ansprüche 1 bis 4 zur Herstellung eines Medikaments für die Behandlung von Nierenerkrankungen.

15. Einsatz einer Verbindung nach einem der Ansprüche 1 bis 4 für die Herstellung eines Medikaments zur Behandlung von Lebererkrankungen.

16. Einsatz einer Verbindung nach einem der Ansprüche 1 bis 4 für die Herstellung einer Medikaments zur Behandlung von Erkrankungen des Nervensystems.

## Revendications

1. Composé **caractérisé par** la formule (I) : 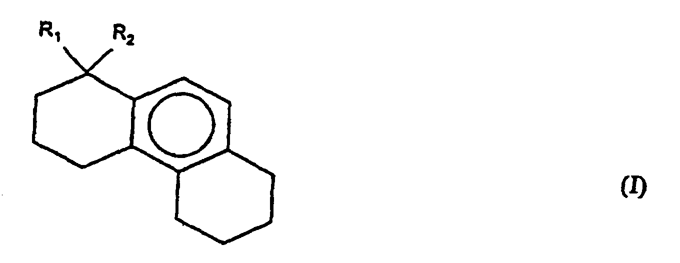 comprenant trois homo-cycles à six atomes de carbone condensés incluant un cycle aromatique central et deux cycles latéraux aliphatiques saturés ou insaturés dans la position méta par rapport audit cycle aromatique central, et dans laquelle les substituants latéraux R₁ et R₂ sont des groupes alkyles avec un nombre d'atomes de carbone de 1 à 4 ; lesdits deux cycles latéraux aliphatiques pouvant comprendre également un ou plusieurs substituants latéraux supplémentaires choisis dans le groupe défini par : =O, -OH, -O-R', -O-R'-OH dans lesquels R' est un groupe aliphatique à chaîne linéaire ou ramifiée, saturé ou insaturé, avec un nombre d'atomes de carbone de 1 à 4.

2. Composé selon la revendication précédente, **caractérisé par** la formule (II) : 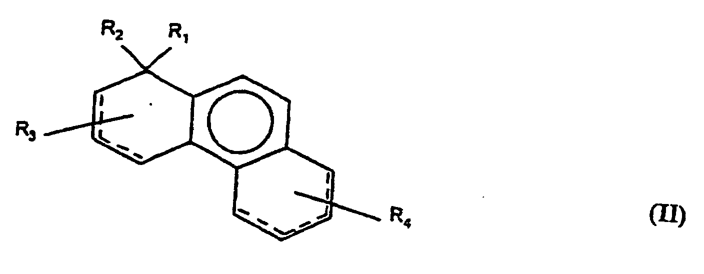 et comprenant au moins un des substituants latéraux R₃ et R₄ et dans laquelle :
- R₁ et R₂ sont des groupes alkyles avec un nombre d'atomes de carbone de 1 à 4 ;
- R₃ et R₄ sont choisis dans le groupe défini par : =O, -OH, -O-R', -O-R'-OH dans lesquels R' est un groupe aliphatique à chaîne linéaire ou ramifiée, saturé ou insaturé, avec un nombre d'atomes de carbone de 1 à 4 ;
- les liaisons représentées par une ligne continue côte à côte avec une ligne pointillée sont des liaisons doubles ou simples.

3. Composé selon la revendication précédente, **caractérisé par** la formule (III) : 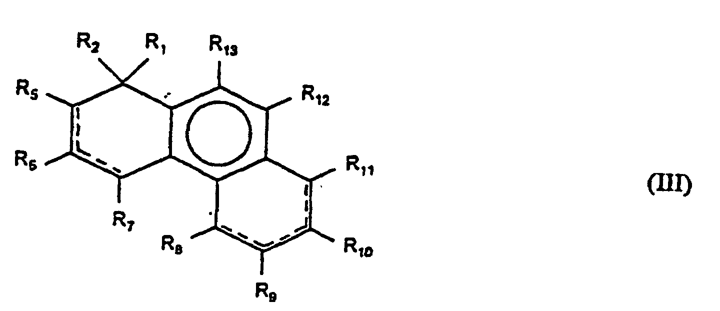 dans laquelle :
- R₁ et R₂ sont des groupes alkyles avec un nombre d'atomes de carbone de 1 à 4 ;
- R₆, R₇, R₈, R₉, R₁₀, R₁₂, R₁₃ sont indépendamment choisis dans le groupe défini par -H, -H₂, =O, -OH, -CH₃ ;
- R₅ et R₁₁ sont choisis dans le groupe défini par : -H, -H₂, =O, -OH, -O-R',
- O-R'-OH dans lesquels R' est un groupe aliphatique à chaîne linéaire ou ramifiée, saturé ou insaturé, avec un nombre d'atomes de carbone de 1 à 4 ;
- les liaisons représentées par une ligne continue côte à côte avec une ligne pointillée sont des liaisons doubles ou simples.

4. Composé selon la revendication précédente, **caractérisé en ce que** R₁ et R₂ sont des groupes méthyles -CH₃ et R₅ et R₁₁ sont choisis dans le groupe défini par -H, -H₂, =O, -OH, -O-CH₃, -O-CH=CH₂, -O-CH=CH₂-CH₃, -O-CH₂-CHOH-CH₃.

5. Composition pharmaceutique pour une utilisation médicale en général, et pour une utilisation en dermatologie, oncologie et dans le traitement des troubles des reins, du foie et du système nerveux ; **caractérisée par le fait qu'**elle comprend le composé selon l'une quelconque des revendications précédentes, en combinaison avec des excipients pharmaceutiquement compatibles.

6. Composition selon la revendication précédente, **caractérisée par le fait qu'**elle comprend également de l'hydroquinone et/ou de l'acide salicylique.

7. Procédé de préparation de compositions pharmaceutiques pour une utilisation médicale en général, et pour une utilisation en dermatologie, oncologie et dans le traitement des troubles des reins, du foie et du système nerveux comprenant le composé selon les revendications 1 à 4 précédentes, **caractérisé par le fait qu'**il comprend les étapes :
- de préparation d'une solution liquide alcoolique d'acide rétinoïque dans un composé alcool ou glycol très fortement en excès par rapport à l'acide rétinoïque ;
- de production d'une réaction d'estérification de l'acide rétinoïque avec ledit composé alcool ou glycol en excès à une température pré-déterminée ;
- consistant à laisser la réaction évoluer pendant un temps pré-déterminé à ladite température pré-déterminée ;
- d'interruption de ladite réaction après ladite durée pré-déterminée, et de refroidissement de ladite réaction.

8. Procédé selon la revendication précédente, **caractérisé en ce que** ladite réaction est produite en amenant ladite solution à ébullition à une température d'environ 187°C.

9. Procédé selon la revendication 7 ou 8, **caractérisé en ce que** ladite réaction est interrompue après environ 60 minutes.

10. Procédé selon la revendication 7 ou 8, **caractérisé en ce que** ladite réaction est interrompue après environ 120 minutes.

11. Composé selon l'une quelconque des revendications 1 à 4 pour une utilisation en tant que médicament.

12. Utilisation d'un composé selon l'une quelconque des revendications 1 à 4 pour la fabrication d'un médicament pour le traitement de maladies dermatologiques.

13. Utilisation d'un composé selon l'une quelconque des revendications 1 à 4 pour la fabrication d'un médicament pour le traitement des cellules tumorales.

14. Utilisation d'un composé selon l'une quelconque des revendications 1 à 4 pour la fabrication d'un médicament pour le traitement des troubles des reins.

15. Utilisation d'un composé selon l'une quelconque des revendications 1 à 4 pour la fabrication d'un médicament pour le traitement des troubles du foie.

16. Utilisation d'un composé selon l'une quelconque des revendications 1 à 4 pour la fabrication d'un médicament pour le traitement des troubles du système nerveux.
